(19) Europäisches Patentamt
European Patent Office
Office européen des brevets

(11) Publication number: 0 247 721 A1

# (12) EUROPEAN PATENT APPLICATION

(21) Application number: 87303486.2

(22) Date of filing: 21.04.87

(51) Int. Cl.⁴: **C 07 D 405/12**, C 07 D 405/14, C 07 D 493/04, A 01 N 43/40

(30) Priority: 28.05.86 GB 8612940

(43) Date of publication of application: 02.12.87 Bulletin 87/49

(84) Designated Contracting States: BE DE FR GB IT NL

(71) Applicant: **THE DOW CHEMICAL COMPANY, 2030 Dow Center Abbott Road P.O. Box 1967, Midland, MI 48640 (US)**

(72) Inventor: **Dennis, Nicholas, 2 Holly Close South Wootton, King's Lynn Norfolk PE30 3JH (GB)**
Inventor: **Burrell, Donald C., 7 Blakeney Fields Great Shefford, Newbury Berkshire (GB)**

(74) Representative: **Raynor, John et al, W.H. Beck, Greener & Co 7 Stone Buildings Lincoln's Inn, London WC2A 3SZ (GB)**

(54) **Alditol derivative esters of triclopyr, processes for their preparation and herbicidal compositions containing them.**

(57) Herbicidal trichlopyr derivatives of the general formula

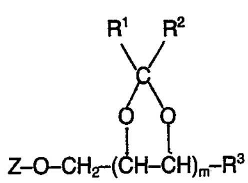
I

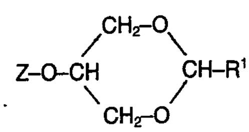
II

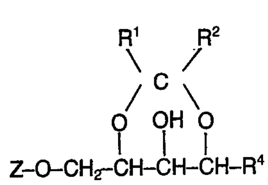
III

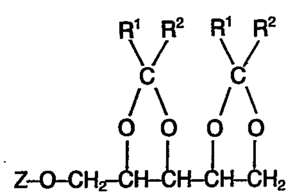
IV

in which Z represents

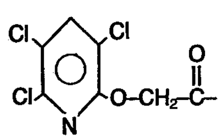
V

$R^1$ and $R^2$ are each independently H, a $C_1$–$C_6$, straight chain or branched alkyl group, or a cycloalkyl group, or a phenyl group optionally mono or di-substituted by any of halogen, $CF_3$, nitro, phenyl, phenoxy, phanylthio, $C_1$–$C_3$ alkylamino, di$C_1$–$C_3$ alkyl amino, $C_1$–$C_3$ alkyl, $C_1$–$C_3$ alkoxy, or $C_1$–$C_3$ alkylthio, or wherein $R^1$ and $R^2$ together are a divalent polymethylene group $(CH_2)_n$, thereby forming a carbocyclic ring having n+1 carbon atoms wherein n is 3, 4, 5 or 6, $R^3$ is H or a group of the formula:

EP 0 247 721 A1

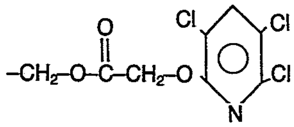

VI

$R^4$ ist H, $CH_2OH$ or a group of formula VI, and m is 1 or 2 are prepared by reacting a compound of the formula YX, wherein Y is a group of formula V and X is hydroxy, halogen or the residue of an alcohol, with a compound of the general formulae I, II, III or IV wherein Y is hydrogen.

# ALDITOL DERIVATIVE ESTERS OF TRICLOPYR, PROCESSES FOR THEIR PREPARATION AND HERBICIDAL COMPOSITIONS CONTAINING THEM

The invention relates to alditol derivative esters of 3,5,6-tricloro-2-pyridyloxyacetic acid, (having the common name triclopyr), processes for their preparation and herbicidal compositions containing them.

Triclopyr is known to possess herbicidal properties, but is also known to cause phytotoxic damage to cereal grains such as wheat and barley when applied postemergently in a herbicidally effective amount upon weeds commonly encountered in cereal grain crop areas. It is also sufficiently volatile to warrant concern for susceptible valuable crops when applied to adjacent land.

The invention provides alditol derivative esters of triclopyr of one of the following general formulae

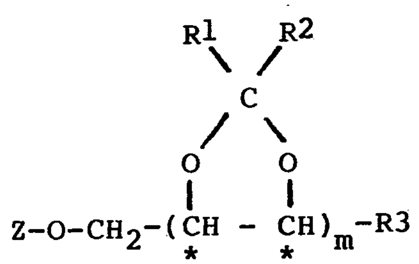

I

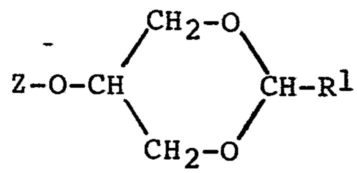

II

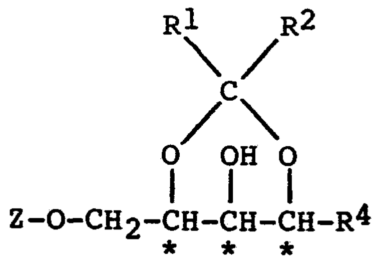

III

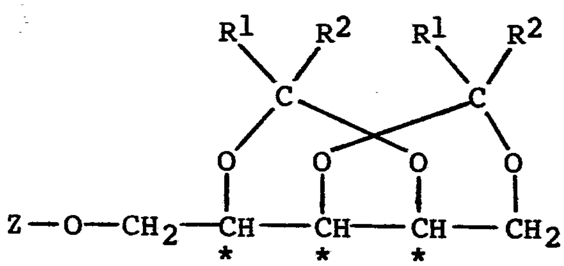

IV

in which Z represents

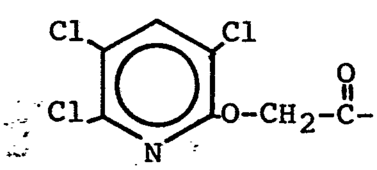

V

$R^1$ and $R^2$ are each independently any sterically comptible combination of H, a $C_1$-$C_6$, straight chain or branched alkyl group, or a cycloalkyl group, or a phenyl group optionally substituted, particularly mon-or di-substituted, by any of halogen, $CF_3$, nitro, phenyl, phenoxy, phenylthio, $C_1$-$C_3$alkylamino, di$C_1$-$C_3$alkyl amino, $C_1$-$C_3$ alkyl, $C_1$-$C_3$ alkoxy, or $C_1$-$C_3$ alkylthio, or wherein $R^1$ and $R^2$ together are a divalent polymethylene group $(CH_2)_n$, thereby forming a carbocyclic ring having n + 1 carbon atoms wherein n is 3,4,5 or 6, $R^3$ is H or a group of the formula:-

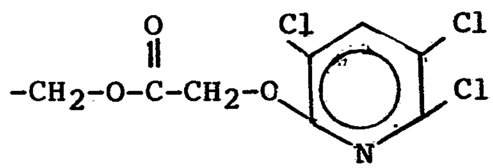

VI

$R^4$ is H, $CH_2OH$ or a group of formula VI, and m is 1 or 2. By halogen is meant any of F, Cl, Br, or $I_2$, although Cl and Br are preferred.

The compounds of the general formulae I, III and IV contain one or more asymmetric carbon atoms, indicated as * in the formulae given above.

Thus the compounds of the invention, having chiral groups, occur as optical isomers. In some cases they also exist in the form of geometrical isomers. Accordingly, the invention also provides the

individual isomers as well as mixtures thereof and it should be understood that the written nomenclature and structural formulae shown in the specification should be taken to include the individual isomers as well as mixtures thereof.

Compounds of the general formulae I, II, III and IV are each of advantage in having substantially lower volatility than triclopyr and in having useful herbicidal activity against one or more significant broad leaf weeds such as fat hen (Chenopodium album) or red dead nettle (Lamium purpureum). They are thus capable of use in controlling such weeds in land adjacent valuable susceptible crops.

Compounds of the general formulae I to IV wherein $R^1$ and $R^2$ are each independently H, $CH_3$, phenyl or phenyl mono or disubstituted by halogen, lower alkyl, lower alkoxy, lower alkylthio, nitro or dialkylamino; m is 1 or 2; $R^3$ is H or a group of formulae VI and $R^4$ is as defined above, are of further advantage in exhibiting useful herbicidal activity against one or more significant broad leaf weeds, such as fat hen (Chenopodium album) or red dead nettle (Lamium purpureum), in some instance to an equal or greater extent than triclopyr or its commercially available ester or sodium or amine salts, whilst exhibiting excellent tolerance by cereal

grains particularly wheat, barley and rice.

Of the compounds according to the invention, those prepared from the acetal of glycerol or from the acetonides of the pentitols, arabitol, adonitol and xylitol are of particular interest, more particularly those prepared from acetonides of arabitol and xylitol.

There should be mentioned especially those compounds in which $R^1$ and $R^2$ are the same or different and each represents H, $CH_3$ or phenyl more especially those in which $R^1$ and $R^2$ are each H or methyl groups, or in which $R^1$ is H and $R^2$ is phenyl. Preferably $R^1$ and $R^2$ are either identical, or else $R^1$ is hydrogen.

Examples of specific compounds having an excellent herbicidal action towards the weeds, fat hen and/or red dead nettle in addition to substantially complete cereal grain tolerance at herbicidally effective dosage rates for the susceptible noxious weeds of the type described, include

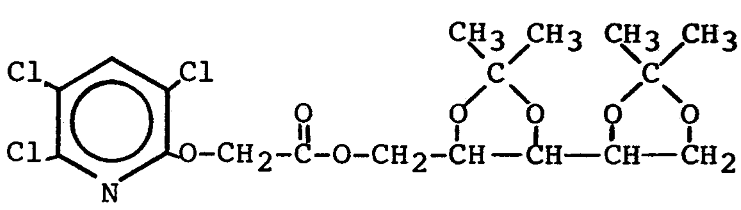

VII

(3,5,6-trichloro-2-pyridyloxyacetyl) 2,3:4,5-di-O-
-(isopropylidene)-D-arabitol;

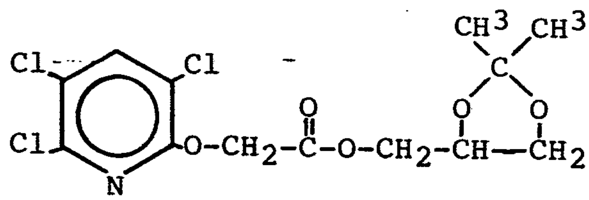

VIII

(3,5,6-trichloro-2-pyridyloxyacetyl) 1,2-(isopropylidene)-glycerol;

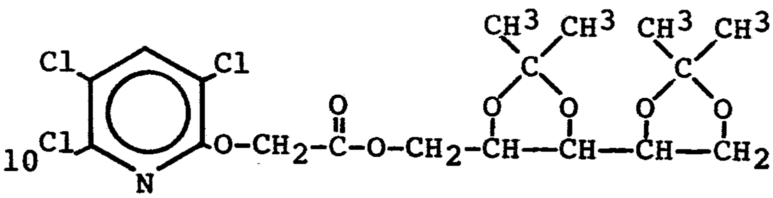

IX

(3,5,6-trichloro-2-pyridyloxyacetyl) 2,3:4,5-di-O--(isopropylidene)-xylitol;

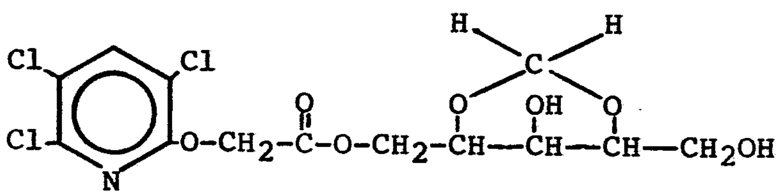

X

1-(3,5,6-trichloro-2-pyridyloxyacetyl) 2,4-(methylene)-adonitol;

XI

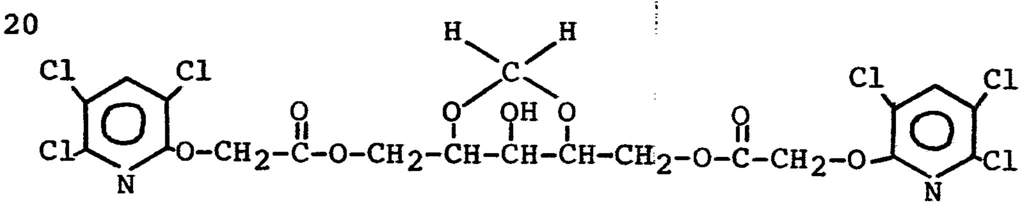

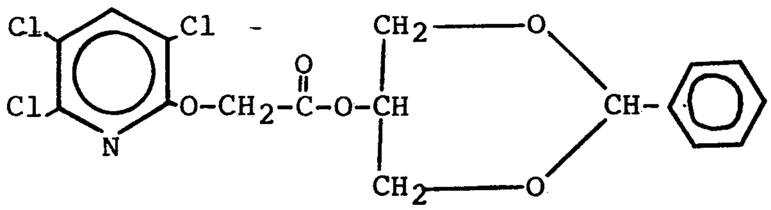

XII

(3,5,6-trichloro-2-pyridyloxyacetyl)-1,3-benzylidene glycerol;

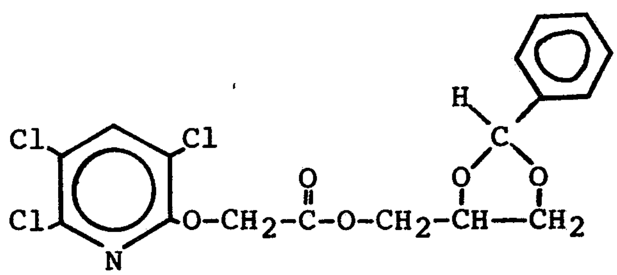

XIII

(3,5,6-trichloro-2-pyridyloxyacetyl)-1,2,-(benzylidene)-glycerol;

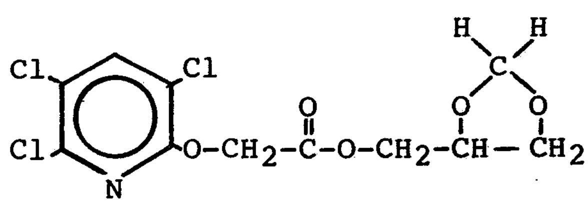

XIV

(3,5,6-trichloro-2-pyridyloxyacetyl)1,2-methylene glycerol; and

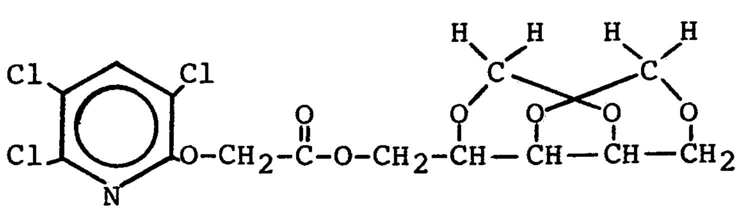

XV

(3,5,6-trichloro-2-pyridyloxyacetyl)-1,3:2,4-di-O-(methylene)-adonitol.

Compounds VII, VIII, IX and X being most preferred on account of their herbicidal activity and broader spectrum.

By substantially complete crop tolerance is meant crop resistance shown by substantial absence of visible foliar damage such as browning or stunting.

Examples of compounds in accordance with the invention, which are not as selective as compounds VII to XV above, but which nevertheless have significant herbicidal activity and low volatility are the following:

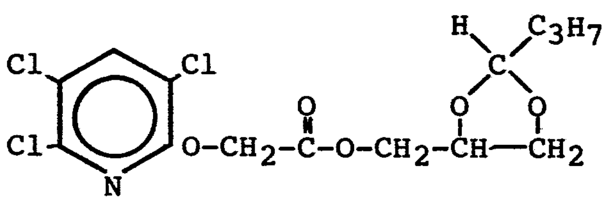

XVI

(3,5,6-trichloro-2-pyridyloxyacetyl)-1,2-(butylidene)-glycerol

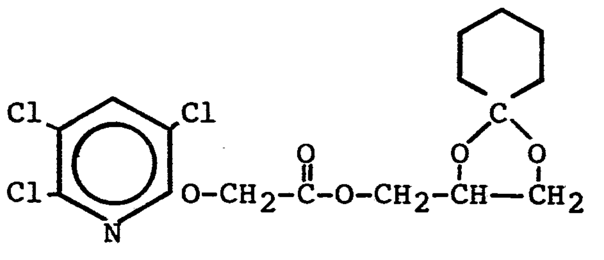

XVII

(3,5,6-trichloro-2-pyridyloxyacetyl); 1,2-(cyclohexylidene)-glycerol

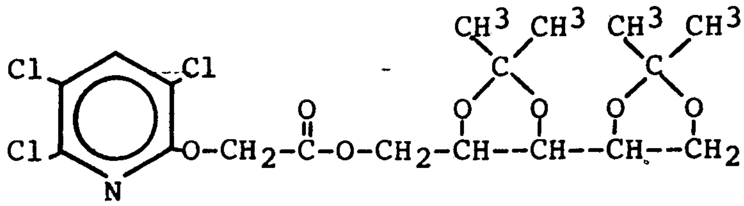

XVIII

(3,5,6-trichloro-2-pyridyloxyacetyl)-2,3:4,5-di-O-(isopropylidene)-L-arabitol

XIX

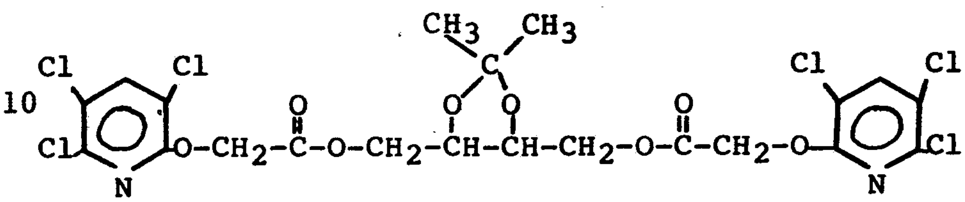

bis-[1,4 (3,5,6-tricloro-2-pyridyloxyacetyl)]-2,3-(isopropylidene)-threitol.

It will be noted that certain of the above compounds, for example compounds VII, IX and XVIII, although different, appear from the structural formulae given to be identical, because they differ only in their sterochemistry.

Compounds in accordance with the invention may generally be prepared by the reaction of triclopyr, or an active halide or ester thereof, i.e. a compound of the formula YX wherein Y is a group of formula V above, and X is hydroxy halogen or the residue of an alcohol, with a compound of the general formulae I, II, III, or IV above, wherein $R^1$, $R^2$, $R^3$, and $R^4$ are as defined above, but Z is hydrogen.

In particular, when X is hydroxy, i.e. when the starting material is trichlopyr itself, the reaction may preferably be carried out in an inert polar solvent, suc as tetrahydrofuran, or acetonitrile in the presence of coupling agent such as N,N'-carbonyldiimidazole, or dicyclohexylcarbodiimide.

The solvent is preferably dried before use, for example using a molecular sieve such as Aldrich type 3A or 4A.

The reaction of the compound of the acid halide of triclopyr (i.e. wherein X is halogen) with the alditol derivative I, II, III, or IV wherein Y is hydrogen may be carried out in a solvent such as acetonitrile, tetrahydrofuran, or dimethylsulfoxide, in the presence of an acid binding agent or scavenger such as dimethylamine, triethylamine, piperidine, pyridine, 4-dimethylaminopyridine, sodium carbonate, or potassium carbonate to achieve the desired condensation. The reaction product may then be filtered, to remove any salt produced, for example the amine hydrochloride salt produced when the scavenger used is an amine. The acid halide of triclopyr may be prepared by the reaction of triclopyr with a suitable halogenating agent, such as $SOCl_2$ or $POCl_3$ in an inert aprotic solvent medium such as toluene, benzene or carbon tetrachloride, to form the acid chloride or other acid halide.

When X is the residue of an alcohol, the starting material is an ester, preferably a $C_1$-$C_6$ alkyl ester such as the methyl ester. The reaction is a transesterification reaction, and is preferably carried out in an aprotic solvent medium in the presence of p-toluenesulphonic acid. The solvent may then be removed.

Accordingly, the present invention provides a process for the preparation of a compound of the general formula I, II, III or IV above by any of the processes described above.

The alditol derivatives (I,II, III and IV, wherein Y is H) may be prepared from the requisite alditol, or polyol and an appropriate aldehyde or ketone in the following manner. The selected ketone or aldehyde and the alditol are brought together in a molar ratio in excess of 2:1 wherein it is anticipated that one dioxo ring will be formed, or 4:1 wherein it is expected two dioxo rings will be formed, in the presence of a small amount of each of copper sulphate and concentrated sulfuric acid and the mixture is stirred, conveniently at room temperature, for from about 5 to about 24 hours or until the reaction is complete. The aldehyde or ketone is preferably used in substantial excess, for example the aldehyde or ketone may be used as the reaction solvent. The

mixture is then neutralized, for example with aqueous ammonia. The copper sulphate and any other insoluble solids are filtered off after which the reaction mixture can be concentrated, e.g., in a rotary evaporator to yield an oily residue, which can be purified for example, by distillation in a khugelrohr apparatus, generally yielding a colourless oil.

Alternatively, the alditol may be reacted with the aldehyde or ketone in the presence of a suitable ion exchange resin, e.g. "DOWEX 50X8-400" (DOWEX is a Trade Mark of The Dow Chemical Company).

Suitable ketones for condensation with the alditol include acetone or di-isopropylketone or other ketones of the structure:

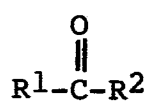

wherein $R^1$ and $R^2$ are as defined above.

Suitable aldehydes are formaldehyde, acetaldehyde, butyraldehyde, benzaldehyde, substituted benzaldehydes, and chloral.

Suitable acid acceptors are, for example, dimethylamine, triethylamine, pyridine, piperidine, 4-methylaminopyridine, sodium carbonate, and potassium carbonate.

When the alditol derivative has two free hydroxyl groups, either mono or bis addition of the trichlopyr (or its derivative) is possible. By addition of the trichlopyr (or its derivative) to the alditol, rather than the reverse, and controlling the amount added to the stoichiometric amount, it is possible to arrange for substantially 1:1 addition, and thereby to prepare the compounds of the invention of formula III, wherein, $R^4$ is $CH_2OH$. Further addition gives the bis-substituted product ($R^4$ is the group of formula IV).

The compounds of the invention may be isolated from the reaction mixture by customary processes, for example, by distilling off the solvent used at normal or reduced pressure, by precipitation with water or by extraction. Generally, a higher degree of purity can be obtained by purification by conventional column-chromatography or flash chromatography, or recrystallization of the product if a solid.

The compounds according to the invention are generally a yellowish gum in character, but some are non-crystalline solids, and some are crystalline solids that are insoluble in water, and to a limited extent, in aliphatic hydrocarbons, such as for example, petroleum ether, hexane, pentane and cyclohexane; and readily soluble in halogenated hydrocarbons, such as, for example, chloroform, methylene chloride and carbon tetrachloride, aromatic

hydrocarbons, such as, for example, benzene, toulene and xylene, ethers, such as, for example, diethyl ether, tetrahydrofuran and dioxan, carboxylic acid nitriles, such as, for example, acetonitrile, ketones, such as, for example, acetone, alcohols, such as, for example, methanol and ethanol, carboxylic acid amides, such as, for example, dimethylformamide, and sulphoxides, such as, for example, dimethyl sulphoxide.

The compounds of the invention generally have been proved to have very low volatility in comparison with the 2-butoxyethyl ester of triclopyr in general too low to show any phytotoxic effects on neighbouring crops.

In the synthesis the active substances are generally obtained as a racemic mixture and can be separated into the optical isomers in a manner known per se, for example, by fractional crystallisation if a solid, or using a chiral chromatographic column or by reaction with a resolving agent such as an alkaloid (e.g. brucine).

Synthesis of optically pure compounds in accordance with the invention may be carried out by using an optically unambiguous D or L alditol as starting material in making the alditol derivative.

The optical isomers, or enantiomers, of the compounds of the invention exhibit differing biological activity, although neither the D or L form

consistently has the greater herbicidal action nor marked difference in selectivity and weed spectrum.

The starting materials for the preparation of the compounds of the invention are known per se or can be manufactured according to processes known per se.

The compounds of the invention may be used alone or in a mixture with one another or with other active substances, if desired, defoliants, plant protecting agents and/or pesticides may be added depending on the desired purpose.

If a broadening of the spectrum of action is intended, other biocides may be added. Examples of suitable herbicidally active mixing partners are carbamic acid derivatives such as phenmedipham, triallate, diallate, and vernolate; dinitrophenol derivatives such as dinoseb and dinoseb acetate; chlorinated aliphatic acids such as trichloroacetic acid and dalapon; amides such as diphenamid and N,N-diallyl-2,2-dichloroacetamide; dipyridilium compounds such as paraquat and diquat; anilides such as propanil, pentanochlor, monalide, propachlor, 2-chloro-2', 6'-diethyl-N-methoxymethylacetanilide; nitriles such as dichlobenil and ioxynil; urea derivatives such as Linuron, chloroxuron, monolinuron, fluometuron and diuron; triazine derivatives such as simazine, atrazine, ametryne, prometryne, desmetryne, methoprotryne, cyanazine and terbumeton; uracil derivatives such as lenacil and bromacil; growth-promoting preparations such as

(2,4,5-trichlorophenoxy) acetic acid, 4-(4-chloro-2-methylphenoxy) butyric acid, 2,3,6-trichlorobenzoic acid, 2-(4-chloro-2-methylphenoxy) propanoic acid and 3,6-dichloro-2-methoxybenzoic acid; aryloxy phenoxy post emergence grass killers, such as gallant; pyridine derivatives such as picloram, clopyralid and fluoroxypyr; and other preparations such as flurecol, 3,4-dichloropropionanilide, trifluralin, bensulide, bifenox, bentazone, pyridate and glyphosate.

Particularly suitable mixing partners are the following: propanil, dichlobenil, ioxynil, diuron, atrazine, cyanazine, bromacil, (2,4,5-trichlorophenoxy) acetic acid, 2,3,6-trichlorobenzoic acid, 2-(4-chloro-2-methylphenoxy) propanoic acid, 3,6-dichloro-2-methoxybenzoic acid, picloram, clopyralid, fluoroxypyr, bentazone, pyridate and glyphosate.

Surprisingly, mixtures according to the invention exhibit a considerably increased action against weeds without damaging the cereal grains treated therewith. Used together with compounds of the invention, the herbicidal action of the mixing partners mentioned is such that, under difficult conditions, such as application in high quantities, they have no specific damaging effect on cereal grains whilst at the same time their spectrum of action against the weed type is broadened.

The ratio of the components in the mixtures according to the invention is preferably approximately from 1:10 to 10:1 more especially from 1:3 to 3:1 by

weight.

Preferred application quantities for selective weed control range from 0.5 to 5.0 kg/ha for the mixing partners listed and from 0.2 to 2 kg/ha for the compounds in accordance with the invention.

It is also advantageous to apply from 0.25 to 5.0 kg/ha of a suitable surface-active substance and other agents that intensify action, for example, non-phytotoxic oils.

Advantageously, the active substances according to the invention or mixtures thereof are used in the form of preparations, for example, powders, sprayable agents, granulates, solutions, emulsions or suspensions, with the addition of liquid and/or solid carriers or diluents and, if desired, wetting agents, adhesion-promoting agents, emulsifiers and/or dispersing agents, but preferably in the form of emulsions and emulsifiable concentrates.

Accordingly, the present invention provides a herbicidal preparation which comprises a compound of the general formula I, II, III or IV, or two or more such compounds, and a suitable carrier.

Suitable liquid carriers are, for example, water, aliphatic and aromatic hydrocarbons, such as, for example, benzene, toluene and xylene, cyclohexanone, isophorone, dimethyl sulphoxide, dimethylformamide, and also mineral oil fractions.

Suitable solid carriers are, for example, mineral earths, for example, tonsil, silica gel, talcum,

kaolin, attaclay, limestone, silica acid and vegetable products, for example, meals.

The preparation advantageously includes a surface-active substance(s). Suitable surface-active substances are, for example, calcium ligninsulphonate, polyoxyethylene alkylphenol ethers, naphthalenesulphonic acides and salts thereof, phenolsulphonic acids and salts thereof, formaldehyde condensates, fatty alcohol sulphates and substituted benzenesulphonic acids and salts thereof.

The proportion of the active substances(s) in the various preparations may vary within wide limits. For example, the preparations may contain from 5 to 95 per cent by weight of active substances, from 95 to 5 per cent by weight of liquid or solid carriers and, if desired, up to 20 per cent by weight of surface-active substances.

The present invention also provides a process of combating weeds, which comprises applying a compound in accordance with the invention, or two or more such compounds; and if desired, other active substances(s), especially a herbicide from the list of mixing partners given above, to a weed or weeds postemergently.

Suitably, the compound(s) are in the form of a herbicidal preparation or preparations comprising

active ingredient and carrier; suitably also when a herbicide mixing-partner is used the compounds are applied as a mixture, for example, in the form of a herbicidal preparation containing the mixture.

The weed or weeds treated may be in a crop area or an area adjacent to a crop area.

The present invention also provides a crop or a crop area treated by the process of the present invention and a crop or other plant in or from a crop area treated by the process of the present invention.

The compounds or preparations may be applied in customary manner, for example, using water as carrier in quantities of spraying liquor of from 100 to 1000 litres/ha. It is possible to use the agents in the so-called Low Volume and Ultra Low Volume processes, and also to apply them in the form of so-called micro-granules.

The following Examples illustrate the invention. Examples 1 to 13 illustrate the preparation of compounds of the invention I. Examples A to C illustrate preparations containing these compounds and Examples I and II illustrate their use.

<u>Example 1</u>

A. <u>2,3:4,5-Di-Isopropylidene-L-Arabitol</u>

A mixture of L-(-)-arabitol (30 g, 0.197 mole) anhydrous copper sulphate (24 g), concentrated sulphuric acid (4 ml) and acetone (600 ml) were

stirred well at room temperature for 18 hours, then neutralised with concentrated aqueous ammonia and filtered. The solution was concentrated using a rotary evaporator to yield a yellow oil as residue. The yellow oil was distilled on a khugelrohr apparatus (110°C) to yield the title compound (35.5 g, 78 per cent) as a colourless oil.

Elemental Analysis:

| | C | H |
|---|---|---|
| Calculated for $C_{11}H_{20}O_5$: | 56.88 | 8.68 |
| Found: | 56.87 | 8.52 |

B. 3,5,6-Trichloro-2-Pyridyloxyacetyl Chloride (Acid Chloride of Triclopyr)

3,5,6-Trichloro-2-pyridyloxyacetic acid (20 g, 0.078 mole was stirred with toluene (46.5 ml). Thionyl chloride (26 ml) was added dropwise to the well-stirred mixture over 15 minutes. The mixture was then heated under reflux (70°C) for 3 hours. The effluent gases ($SO_2$ + HCl) were absorbed into aqueous 5N sodium hydroxide. The cooled reaction mixture was filtered and evaporated its yield to the title compound (20.9 g, 98 per cent) as a thick dark brown oil.

C. <u>(3,5,6-Trichloro-2-pyridyloxyacetyl)-2,3:4,5-Di-O-(Isopropylidene)-L-Arabitol (Compound XVIII)</u>

2,3:4,5-Di-O-isopropylidene-l-arabitol (6 g, 0.026 mole) was dissolved in acetonitrile (30 ml) and triethylamine (3 g) added. 3,5,6-Trichloro-2-pyridyloxyacetyl chloride (7.1 g, 0.026 mole, in acetonitrile (20 ml) was added dropwise with stirring over 15-20 minutes. The mixture was stirred for a further 3 hours and the reaction followed by thin layer chromatography (TLC) of small samples of the reaction mixture.

On cooling, the solid precipitate was removed by filtration and the filtrate concentrated on the rotary evaporator. The oily residue was taken up in dichloromethane (100 mls) and washed thoroughly with water to remove any triethylamine hydrochloride remaining. After drying with sodium sulphate, evaporation of the dichloromethane layer gave a thick dark brown oil as crude product.

This was eluted over a neutral alumina column using benzene as the solvent to yield the title compound (6.9 g, 57 per cent) as a pale yellow viscous gum.

Elemental Analysis

| | C | H | N |
|---|---|---|---|
| Calculated for $C_{18}H_{22}Cl_3NO_7$: | 45.93 | 4.71 | 2.98 |
| Found: | 46.10 | 4.55 | 2.98 |

Example 2

A. 2,3:4,5-Di-O-Isopropylidene-D-Arabitol

A mixture of D-(+)-arabitol (30 g, 0.197 mole) anhydrous copper sulphate (24 g), concentrated sulphuric acid (4 ml), and acetone (600 ml) were stirred well at room temperature for 18 hours, then neutralised with concentrated aqueous ammonia and filtered. The solution was concentrated using a rotary evaporator to yield a blue-green oil as residue.

The blue-green oil was distilled on a khugelrohr apparatus (110°) to yield the title compound (38.0 g, 83 per cent) as a colourless oil.

B. 3,5,6-Trichloro-2-Pyridyloxyacetyl-2,3:4,5-Di-O-(Isopropylidene)-D-Arabitol(Compound VII)

The procedure of Example 1-C was repeated except there was used the above described 2,3:4,5-di-O-isopropylidene-L-arabitol in place of the D compound.

The title compound was obtained (8.3 g, 68 per cent) as a pale yellow viscous gum.

Elemental Analysis:

| | C | H | N |
|---|---|---|---|
| Calculated for $C_{18}H_{22}Cl_3NO_7$: | 45.93 | 4.71 | 2.98 |
| Found: | 46.46 | 4.72 | 2.80 |

Example 3

3,5,6-Trichloro-2-Pyridyloxyacetyl -1,2-(Isopropylidene)-Glycerol (Compound VIII)

N,N',-carbonyldiimidazole (8.75 g, 0.054 mole) was added to 3,5,6-trichloro-2-pyridyloxyacetic acid (13.8 g, 0.054 mole) in dry tetrahydrofuran (120 ml) at room temperature with constant stirring. When evolution of carbon dioxide ceased - the mixture was treated with 1,2-isopropylidene glycerol (7.1 g, 0.054 mole) and stirred for a further hour before leaving to stand overnight. The reaction was followed by T.L.C.

The solid precipitate was removed by filtration and the filtrate concentrated on the rotary evaporator. The oily residue was taken up in dichloromethane (100 ml) and washed thoroughly with water. After drying with sodium sulphate, evaporation gave a pale, brown, oil. This was eluted with benzene over a neutral alumina column to yield the product as a pale yellow oil (8.3 g, 41.5 per cent).

Elemental Analysis:

| | C | H | N |
|---|---|---|---|
| Calculated for $C_{13}H_{22}Cl_3NO_7$: | 42.13 | 3.81 | 3.78 |
| Found: | 42.07 | 3.75 | 3.66 |

After a short period of standing, the oil crystallized out. Recrystallization from hexane

yielded the title compound (6.9 g; 34.5 per cent) as a white crystalline solid, m.p. 73°-74°C.

Example 4

1-(3,5,6-Trichloro-2-pyridyloxyacetyl) -2,4-(Methylene)-Adonitol (Compound X)

N,N'Carbonyloiimidazole (3.47g, 0.0195 mole) was added to 3,5,6-trichloro-2-pyridyloxyacetic acid (5.0g, 0.0195 mole) in dry tetrahydrofuran (70 ml) at room temperature with constance stirring. When evolution of carbon dioxide ceased, the mixture was added drowise to a solution of 2,4-methylene adonitol (3.2g, 0.0195 mole) in dry tetrahydrofuran (70 ml). After stirring for an hour, the reaction was allowed to stand overnight.

The solid pricipitate was removed by filtration and the filtrate concentrated on a rotary evaporator. The oil residue was taken up in dichloromethane (100 ml) and washed thoroughly with water. After drying with sodium pulphate, evaporation gave a gum. This product was eluted with ethyl acetate and hexane mixtures (starting with 1:4 and ending with 100% ethyl acetate) over a silica column to yield the product as a white solid, m.p. 123.6°C. (7.0g, 79.5 per cent).

| Elemental Analysis: | C | H | N |
| --- | --- | --- | --- |
| Calculated for $C_{13}H_{22}Cl_3NO_7$: | 38.78 | 3.51 | 3.48 |
| Found: | 38.99 | 3.47 | 3.54 |

## Examples 5 to 13

The following compounds were prepared in an analogous manner:

| Example No. | Name of Compound | M.pt. if solid (°C) |
|---|---|---|
| 5 | (3,5,6-trichloro-2-pyridyloxyacetyl) -2,3:4,5-di-O-(isopropylidene) -xylitol; (Compound IX) | oil |
| 6 | (3,5,6-trichloro-2-pyridyloxyacetyl) -1,3-(benzylidene)- glycerol; (Compound XII) | 97.5 |
| 7 | (3,5,6-trichloro-2-pyridyloxyacetyl) -1,2-(benzylidene)-glycerol; (Compound XIII) | oil |
| 8 | (3,5,6-trichloro-2-pyridyloxyacetyl) -1,2-(methylene)-glycerol; (Compound XIV) | 120.5 |
| 9 | (3,5,6-trichloro-2-pyridyloxyacetyl) -1,3:2,4-di-O-(methylene)-adonitol (Compound XV) | 126.2 |

| Example No. | Name of Compound | M.pt if solid (°C) |
|---|---|---|
| 10 | (3,5,6-trichloro-2-pyridyloxyacetyl) -1,2-(butylidene)- glycerol; (Compound XVI) | oil |
| 11 | (3,5,6-trichloro-2-pyridyloxyacetyl) -1,2-(cyclohexylidene)-glycerol; (Compound XVII) | oil |
| 12 | (3,5,6-trichloro-2-pyridyloxyacetyl) -2,3:4,5-di-O-(isopropylidene)-L-arabitol; (Compound XVIII) | oil |
| 13 | bis-[1,4-(3,5,6-trichloro-2-pyridyloxyacetyl)]-2,3-(isopropylidene)-threitol; (Compound XIX) | oil |

## FORMULATION EXAMPLES

The following Examples A to C are illustrations of typical formulations in which the compounds of the invention may be utilised.

A. Wettable Powder

a) 40 per cent by weight of active substance

25 per cent by weight of clay minerals

20 per cent by weight of colloidal silica

10 per cent by weight of cellulose pitch

5 per cent by weight of surfactant based on a mixture of the calcium salt of lignin-sulphonic acid and alkylphenol polyglycol ethers

b) 25 per cent by weight of active substance

60 per cent by weight of kaolin

10 per cent by weight of colloidal silica

5 per cent by weight of surfactant based on the sodium salt of N-methyl-N-oleyltaurine and the calcium salt of lignin-sulphonic acid

c) 10 per cent by weight of active substance

60 per cent by weight of clay minerals

15 per cent by weight of cellulose pitch

10 per cent by weight of surfactants based on the sodium salt of N-methyl-N-oleyltaurine and the calcium salt of lignin-sulphonic acid

B. Paste

45 per cent by weight of active substance

5 per cent by weight of sodium aluminium silicate

15 per cent by weight of cetyl polyglycol ether with 8 moles of ethylene oxide

2 per cent by weight of spindle oil

10 per cent by weight of polyethylene glycol

23 per cent by weight of water

C. Emulsion Concentrate

a) 20 per cent by weight of active substance

15 per cent bv weight of cyclohenaxone

55 per cent by weight of xylene

5 per cent by weight of a mixture of nonylphenyl polyoxyethylenes and calcium dodecylbenzenesulphonate

b) 10 per cent by weight of active substance

6 per cent by weight of cyclohexanone

36 per cent by weight of xylene

12 per cent by weight of a mixture of nonylphenyl polyoxyethylenes and calcium dodecylbenzenesulphonate

36 per cent by weight of mineral oil having a high paraffin content.

The following Examples illustrate possible uses of the compounds and mixtures according to the invention which ere used in the form of the above-mentioned preparations.

Example I

Glasshouse tests were carried out to compare the herbicidal efficacy of a postemergence application of the esters of triclopyr prepared in Examples 1 to 13 against neat triclopyr ethoxybutyl ester and a commercially available triclopyr ethoxybutyl ester formulation ("GARLON-4" GARLON-4 is a Trade Mark of The Dow Chemical Company).

The esters of triclopyr in accordance with the invention were prepared by coupling the corresponding free acid with the appropriate alditol derivative as described in examples 1 to 4 in the presence of N,N'-carbonyldiimidazole. The chiral D-and L-ester herbicides were produced by using the corresponding D- and L- arabitol acetonides prepared from D- and L- alcohols. The esters were purified by column chromatography on neutral alumina.

The new compounds were characterizerd by Infrared Spectroscopy, Nuclear Magnetic Resonance Spectroscopy and Elemental Analysis and had the structures given above.

Method

All plants were cultivated in the glasshouse, to a true 2-4 leaf stage, in a mixture of 70 per cent peat, 20 per cent sharp sand and 10 per cent potting grit. Then they were placed in a cooler area to harden off, at temperatures ranging from 5°C at night to 15°C during the day.

Concentrates of the respective new compounds, GARLON-4, and neat trichlopyr ethoxybutyl ester were made up as aqueous compositions in water utilizing 25 per cent acetone and 0.25 per cent of a surfactant (Triton X-100), to disperse the active ingredients (TRITON X-100 is a Trade Mark of Rohm and Haas).

The concentrates were then further diluted with water containing 0.25 per cent of Triton X-100, to produce sprayable formulations of the desired concentration.

All formulations were applied to the plants using a MARDRIVE overhead sprayer set at 30 psi, delivering 300 l/ha. Plants were returned to the glasshouse after treatment and were subjected to temperatures ranging between 15°C at night to 20°C during the day. The compounds were applied at rates of 960, 480 and 240 grams active ingredient (calculated as trichlopyr free acid) per hectare (g ai/ha).

Application of the formulations was made to the following plants postemergently.

Weeds:

Cleaver (*Galium aparine*)

Fat hen (*Chenopodium album*)

Red Dead Nettle (*Lamium purpureum*)

Pansy (*Viola arvensis*)

Crops:

Barley (Golden Promise)

Wheat (Norman)

ASSESSMENTS

A 0-5 scale was used to assess all tests:

0 = Normal healthy plants

5 = Dead

Efficacy results at indicated times and application rates are summarised in Table 1.

Example II

Various of the alditol ester according to the invention were tested for their volatile vapour effects on adjacent sensitive crops, and compared with technical trichlopyr ethoxybutyl ester, and GARLON-4.

A wind tunnel was constructed using a glasshouse constructed of transparent rigid sheets secured to a tubular aluminium frame 0.43m high, 0.84m wide and 3.05m in length.

An airflow was introduced into one end of the tunnel using a Secomak (Model 573) fan giving an airflow of 3/4 M.P.H. from a manifold and vented to the atmosphere at the opposite end of the tunnel.

Test compounds were sprayed over plants representative of the field target eg. turf, at normal field rates and volumes (typically 960g/ha calculated as trichlopyr free acid). The target was allowed to dry and was then introduced into the upwind end of the tunnel. Air was passed over the target for a period

of 24 hours and vented to the atmosphere outside the glasshouse.

Various sensitive plants (cotton, barley, pinto beans, and rape) were placed at varying distances downwind of the target for the 24 hour period.

After 24 hours the sensitive plants were removed from the tunnel and grown on a glasshouse bench for a further period of 14 days.

Plants exposed at distances of 0, 1, 2, 3, 4, 5, 6, and 7 ft. (0, 0.3, 0.6, 0.9, 1.2, 1.5, 1.8 and 2.1 metres) from the target were assessed visually immediately after removal from the tunnel, on a scale of 0 to 5, where 0 = normal healthy plants, and 5 = dead.

The results are shown in Table 2. Further observation of the plants over the 14 day period after removal from the tunnel showed no significant change in their score immediately after removal from the tunnel.

Tables 1 and 2 illustrate the substantially improved selectivity of certain compounds of the invention, as compared with the trichlopyr ethoxbutyl ester, and their lower deleterious effects on adjacent crops.

TABLE 1

ACTIVITY ASSESSMENT OF ARABITOL DERIVATIVE ESTERS OF TRICLOPYR

| TEST HERBICIDE | RATE g/ha | CLEAVER DAYS | | | RED DEAD NETTLE DAYS | | | PANSY DAYS | | | FAT HEN DAYS | | | WHEAT DAYS | | | WHEAT DAYS | | |
|---|---|---|---|---|---|---|---|---|---|---|---|---|---|---|---|---|---|---|---|
| | | 4 | 13 | 19 | 4 | 13 | 19 | 4 | 13 | 19 | 4 | 13 | 19 | 4 | 13 | 19 | 4 | 13 | 19 |
| Trichlopyr Ethoxybutyl Ester (technical) | 960 | 2 | 2 | 0 | 2 | 3 | 3 | 2 | 2 | 2 | 4 | 4 | 4 | 0 | 0 | 0 | 2 | 3 | 3 |
| | 480 | 2 | 2 | 0 | 2 | 3 | 3 | 2 | 1 | 2 | 3 | 3 | 4 | 0 | 0 | 0 | 2 | 3 | 3 |
| | 240 | 0 | 2 | 0 | 2 | 3 | 3 | 2 | 1 | 2 | 3 | 3 | 4 | 0 | 0 | 0 | 2 | 3 | 2 |
| (GARLON-4) | 960 | 3 | 3 | 2 | 3 | 4 | 5 | 3 | 4 | 4 | 4 | 5 | 5 | 0 | 0 | 0 | 1 | 3 | 3 |
| | 480 | 3 | 3 | 2 | 3 | 4 | 4 | 3 | 4 | 4 | 4 | 4 | 4 | 0 | 0 | 0 | 1 | 3 | 3 |
| | 240 | 2 | 2 | 0 | 2 | 4 | 3 | 2 | 3 | 3 | 3 | 4 | 4 | 0 | 0 | 0 | 0 | 2 | 2 |
| Compound VII | 960 | 3 | 3 | 1 | 3 | 4 | 4 | 3 | 3 | 3 | 4 | 4 | 4 | 0 | 0 | - | 0 | 0 | - |
| | 480 | 3 | 2 | 0 | 3 | 3 | 4 | 3 | 3 | 3 | 4 | 4 | 4 | 0 | 0 | - | 0 | 0 | - |
| | 240 | 2 | 2 | 0 | 2 | 3 | 3 | 3 | 3 | 2 | 4 | 4 | 4 | 0 | 0 | - | 0 | 0 | - |
| Compound VIII | 960 | 2 | 0 | 0 | 2 | 2 | 4 | 1 | 3 | 2 | 4 | 4 | 4 | 0 | 0 | 0 | 0 | 0 | 0 |
| | 480 | 1 | 0 | 0 | 0 | 1 | 2 | 1 | 2 | 1 | 4 | 4 | 4 | 0 | 0 | 0 | 0 | 0 | 0 |
| | 240 | 0 | 0 | 0 | 0 | 1 | 2 | 0 | 2 | 0 | 4 | 4 | 4 | 0 | 0 | 0 | 0 | 0 | 0 |
| Compound IX | 960 | 0 | 0 | 0 | 0 | 2 | 3 | 1 | 1 | 0 | 3 | 4 | 4 | 0 | 0 | 0 | 0 | 0 | 0 |
| | 480 | 0 | 0 | 0 | 0 | 2 | 2 | 1 | 1 | 0 | 3 | 3 | 4 | 0 | 0 | 0 | 0 | 0 | 0 |
| | 240 | 0 | 0 | 0 | 0 | 2 | 2 | 1 | 1 | 0 | 2 | 3 | 4 | 0 | 0 | 0 | 0 | 0 | 0 |
| Compound X | 960 | 1 | 2 | 2 | 1 | 2 | 2 | 2 | 2 | 2 | 3 | 4 | 4 | 0 | 0 | 0 | 2 | 1 | 1 |
| | 480 | - | - | - | - | - | - | 2 | 2 | 1 | 3 | 4 | 3 | - | - | - | 2 | 1 | 1 |
| | 240 | - | - | - | - | - | - | 2 | 2 | 1 | 2 | 3 | 3 | - | - | - | 2 | 1 | 1 |

TABLE 1 (Continued)

ACTIVITY ASSESSMENT OF ARABITOL DERIVATIVE ESTERS OF TRICLOPYR

| TEST HERBICIDE | RATE g/ha | CLEAVER DAYS 4 | CLEAVER DAYS 13 | CLEAVER DAYS 19 | RED DEAD NETTLE DAYS 4 | RED DEAD NETTLE DAYS 13 | RED DEAD NETTLE DAYS 19 | PANSY DAYS 4 | PANSY DAYS 13 | PANSY DAYS 19 | FAT HEN DAYS 4 | FAT HEN DAYS 13 | FAT HEN DAYS 19 | WHEAT DAYS 4 | WHEAT DAYS 13 | WHEAT DAYS 19 | WHEAT DAYS 4 | WHEAT DAYS 13 | WHEAT DAYS 19 |
|---|---|---|---|---|---|---|---|---|---|---|---|---|---|---|---|---|---|---|---|
| Compound XI | 960 | 0 | 0 | 0 | 0 | 0 | 0 | 1 | 2 | 2 | 3 | 4 | 3 | 0 | 0 | 0 | 2 | 1 | 0 |
| | 480 | - | - | - | - | - | - | 1 | 1 | 1 | 2 | 3 | 2 | - | - | - | 1 | 1 | 0 |
| | 240 | - | - | - | - | - | - | 0 | 0 | 0 | 2 | 3 | 2 | - | - | - | 1 | 1 | 0 |
| Compound XII | 960 | 2 | 2 | 3 | 1 | 0 | 1 | 0 | 1 | 1 | 2 | 3 | 3 | 0 | 0 | 0 | 2 | 0 | 0 |
| | 480 | - | - | - | 1 | 0 | 0 | 0 | 1 | 0 | 2 | 3 | 3 | - | - | - | 1 | 0 | 0 |
| | 240 | - | - | - | 1 | 0 | 0 | 0 | 1 | 0 | 2 | 3 | 3 | - | - | - | 1 | 0 | 0 |
| Compound XIII | 960 | 2 | 3 | 4 | 2 | 3 | 3 | 3 | 2 | 3 | 3 | 3 | 3 | 0 | 0 | 0 | 3 | 3 | 3 |
| | 480 | - | - | - | - | - | - | - | - | - | - | - | - | - | - | - | - | - | - |
| | 240 | - | - | - | - | - | - | - | - | - | - | - | - | - | - | - | - | - | - |
| Compound XIV | 960 | 2 | 0 | 0 | 1 | 3 | 3 | 1 | 3 | 2 | 2 | 3 | 3 | 0 | 0 | 0 | 0 | 1 | 1 |
| | 480 | 2 | 0 | 0 | 0 | 2 | 2 | 1 | 1 | 0 | 2 | 3 | 3 | 0 | 0 | 0 | 0 | 0 | 0 |
| | 240 | 1 | 0 | 0 | 0 | 2 | 2 | 1 | 1 | 0 | 2 | 3 | 3 | 0 | 0 | 0 | 0 | 0 | 0 |

TABLE 1 (Continued)

ACTIVITY ASSESSMENT OF ARABITOL DERIVATIVE ESTERS OF TRICLOPYR

| TEST HERBICIDE | RATE g/ha | CLEAVER DAYS 4 13 19 | RED DEAD NETTLE DAYS 4 13 19 | PANSY DAYS 4 13 19 | FAT HEN DAYS 4 13 19 | WHEAT DAYS 4 13 19 | WHEAT DAYS 4 13 19 |
|---|---|---|---|---|---|---|---|
| Compound XV | 960 | 1 1 2 | 0 0 0 | 1 0 1 | 3 3 2 | 0 0 0 | 1 0 0 |
| | 480 | - - - | - - - | 1 0 0 | 2 2 2 | - - - | 1 0 0 |
| | 240 | - - - | - - - | 0 0 0 | 2 2 0 | - - - | 0 0 0 |
| Compound XVI | 960 | 2 3 4 | - - - | 3 2 4 | 3 3 3 | 0 0 0 | 3 3 4 |
| | 480 | 1 2 2 | - - - | 3 2 4 | 3 3 3 | 0 0 0 | 2 3 3 |
| Compound XVII | 960 | 2 3 4 | - - - | 3 3 4 | 3 4 4 | 0 0 0 | 3 3 3 |
| | 480 | 2 3 3 | - - - | 3 3 4 | 3 3 3 | 0 0 0 | 3 3 3 |
| Compound XVIII | 960 | 3 3 2 | 2 4 4 | 3 3 4 | 4 4 5 | 0 0 - | 0 0 - |
| | 480 | 2 3 2 | 3 4 4 | 3 2 2 | 4 4 5 | 0 0 - | 0 0 - |
| | 240 | 2 2 0 | 3 3 3 | 2 2 0 | 4 4 5 | 0 0 - | 0 0 - |
| Compound XIX | 960 | 1 1 1 | - - - | 2 0 1 | 3 3 3 | 0 0 0 | 2 2 2 |
| | 480 | 1 0 1 | - - - | 2 0 1 | 3 3 3 | 0 0 0 | 2 2 1 |

TABLE 2

ASSESSMENTS OF VARIOUS ARABITOL DERIVATIVE ESTERS OF TRICLOPYR

VOLATILITY TESTS

| Test Herbicide | COTTON Distance in Ft 0 1 2 3 4 5 6 7 | BARLEY Distance in Ft 0 1 2 3 4 5 6 7 | PINTO BEANS Distance in Ft 0 1 2 3 4 5 6 7 | RAPE Distance in Ft 0 1 2 3 4 5 6 7 |
|---|---|---|---|---|
| Compound XVIII | 0 0 0 0 0 0 0 0 | 0 0 0 0 0 0 0 0 | 0 0 0 0 0 0 0 0 | 0 0 0 0 0 0 0 0 |
| Compound VIII | 0 0 0 0 0 0 0 0 | 0 0 0 0 0 0 0 0 | 0 0 0 0 0 0 0 0 | 0 0 0 0 0 0 0 0 |
| Compound XIV | 0 0 0 0 0 0 0 0 | 0 0 0 0 0 0 0 0 | 0 0 0 0 0 0 0 0 | 0 0 0 0 0 0 0 0 |
| Compound IX | 0 0 0 0 0 0 0 0 | 0 0 0 0 0 0 0 0 | 0 0 0 0 0 0 0 0 | 0 0 0 0 0 0 0 0 |
| Trichlopyr ethoxybutyl ester | 1 1 1 1 1 1 1 1 | 2 2 2 2 2 1 1 0 | 3 3 2 2 2 1 1 1 | 3 3 3 3 2 2 2 2 |
| GARLON-4 | 3 3 3 3 3 2 2 2 | 2 2 1 1 0 0 0 0 | 3 3 3 2 2 2 2 2 | 2 2 2 1 2 2 2 1 |

Assessment Scale 0 - 5: 0 = Normal healthy plants; 5 = dead

CLAIMS

1. A compound of the general formula

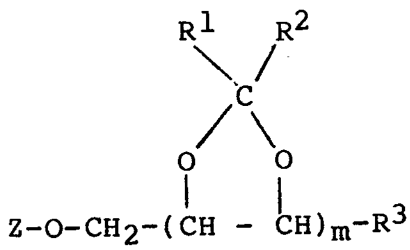

I

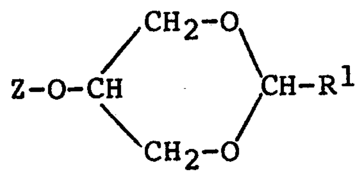

II

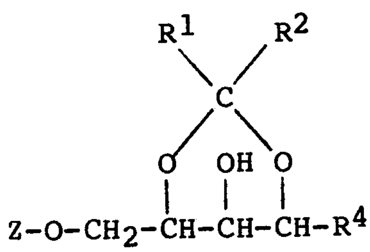

III

or

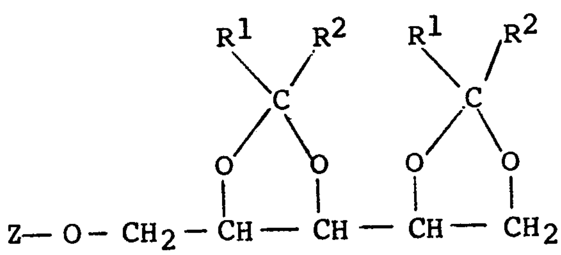

IV

in which Z represents

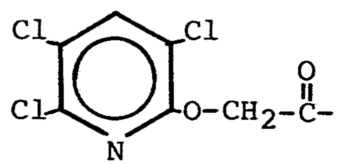

V

$R^1$ and $R^2$ are each independently H, a $C_1$ - $C_6$, straight chain or branched alkyl group, or a cycloalkyl group, or a phenyl group optionally mono or di-substituted by any of halogen, $CF_3$, nitro, phenyl, phenoxy, phenylthio, $C_1$-$C_3$ alkylamino, di$C_1$-$C_3$ alkyl amino, $C_1$-$C_3$ alkyl, $C_1$-$C_3$ alkoxy, or $C_1$-$C_3$ alkylthio, or wherein $R^1$ and $R^2$ together are a divalent polymethylene group $(CH_2)_n$, thereby forming a carbocyclic ring having n + 1 carbon atoms wherein n is 3,4,5 or 6, $R^3$ is H or a group of the formula:-

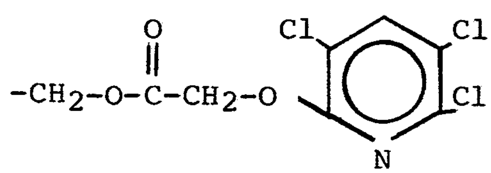

VI

$R^4$ is H, $CH_2OH$ or a group of formula VI, and m is 1 or 2.

2. A compound as claimed in Claim 1, wherein $R^1$ and $R^2$ are each independently H, $CH_3$, phenyl or phenyl mono or disubstituted by halogen, lower alkyl, lower alkoxy, lower alkylthio, nitro or dialkylamino; m is 1 or 2; $R^3$ is H or a group of formulae VI and $R^4$ is as defined in Claim 1.

3. A compound as claimed in Claim 1, wherein $R^1$ and $R^2$ are the same, or $R^1$ is hydrogen.

4. A compound as claimed in any one of the preceding claims, wherein $R^1$ and $R^2$ are the same or different and each represents H, $CH_3$ or phenyl.

5. A compound as claimed in Claim 4, wherein $R^1$ and $R^2$ are each hydrogen or methyl.

6. A compound as in any of Claims 1-7, wherein R and $R^1$ are each methyl.

7. (3,5,6-trichloro-2-pyridyloxyacetyl) 2,3:4,5-di-O-(isopropylidene)-D-arabitol; (3,5,6-trichloro-2-pyridyloxyacetyl) 1,2-(isopropylidene)-glycerol; (3,5,6-trichloro-2-pyridyloxyacetyl) 2,3:4,5-di-O-(isopropylidene)-xylitol; or (3,5,6-trichloro-2-pyridyloxyacetyl) -2,4-(methylene)-adonitol.

8. A process for the preparation of a compound as claimed in Claim 1 which process comprises reacting a compound of the formula YX, wherein Y is a group of

formula V as defined in Claim 1 and X is hydroxy, halogen or the residue of an alcohol, with a compound of the general formulae I, II, III or IV as defined in Claim 1 wherein $R^1$, R2, R3, and R4 are as defined in Claim 1, but Z is hydrogen.

9. A process as claimed in Claim 8, wherein X is hydroxy, and the reaction is carried out in the presence of an acid coupling agent, wherein X is halogen, and the reaction is carried out in the presence of an acid binding agent, or wherein X is the residue of an alcohol, and the reaction is carried out in the presence of p-toluene sulphonic acid.

10. A herbicidal composition, which comprises a compound as claimed in any one of Claims 1 to 7 or prepared by a process as claimed in Claim 8 or Claim 9 together with a carrier or additional active ingredient.

11. A composition as claimed in Claim 10, wherein the additional active ingredient is phenmedipham, triallate, diallate, vernolate, dinitrophenol dinoseb, dinoseb acetate, trichloroacetic acid, dalapon, diphenamid, N,N-diallyl-2,2-chloroacetamide, paraquat, diquat, propanil, solan, monalide, propachlor, 2-chloro-2',6'-diethyl-N-methoxymethylacetanilide, dichlobenil, ioxynil, linuron, chloroxuron, monolinuron, fluometuron, diuron, simazine, atrazine,

ametryne, prometryne, desmetryne, methoprotryne, desmetryne, methoprotryne, cyanazine terbumeton, lenacil, bromacil, (2,4-dichlorophenoxy) acetic acid, 2,4,5-trichlorophenoxy) acetic acid, 4-(4,chloro-2-methylphenoxy) butyric acid, 2,3,6-trichlorobenzoic acid, 2-(4-chloro-2-methylphenoxy) propanoic acid, 3,6-dichloro-2-methoxybenzoic acid), picloram, clopyralid, fluoroxypr, flurenol, 3,4-dichloropropionanilide, trifluralin, bensulide, bentazone, pyridate or glyphosate.

12. A process for combating weeds, which comprises applying a compound as claimed in any one of Claims 1 to 7 or prepared by a process as claimed in Claim 8 or Claim 9, or a composition as claimed in Claim 10 or Claim 11, to a weed or weeds in at least a herbicidally effective amount.

13. A process as claimed in Claim 12 wherein the said compound is applied in a total amount of from 0.2 to 2 kg/ha.

14. A process as claimed in Claim 13, wherein the said compound is applied in a total amount of from 0.5 to 1.2 kg/ha.

15. A process as claimed in any one of Claims 12 to 14, wherein the said compound is applied to a crop area or an area adjacent to a crop area.

16. A process as claimed in Claim 15, wherein the crop area treated is a cereal grain area.

17. A process as claimed in Claim 16, wherein the crop area treated is a wheat or barley area.

18. A process as claimed in any one of Claims 13 to 17, wherein the weeds comprise Galium aparine, Chenopodium album, Lamium purpureum, or Viola arvensis.

European Patent Office

# EUROPEAN SEARCH REPORT

0247721
Application number

EP 87303486.2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | CHEMICAL ABSTRACTS, vol. 101, no. 13, September 24, 1984, Columbus, Ohio, USA<br>MITSUI TOATSU CHEMICALS, INC. "Tetrahydrofuryl aryloxyacetates as herbicides."<br>page 637, column 2, abstract no. 110716z<br>& Jpn. Kokaí Tokkyo Koho JP 59 70,680 [84 70,680]<br>-- | 1,10 | C 07 D 405/12<br>C 07 D 405/14<br>C 07 D 493/04<br>A 01 N 43/40 |
| A | CHEMICAL ABSTRACTS, vol. 90, no. 17, April 23, 1979, Columbus, Ohio, USA<br>ABDALLA, M.; ESSAWY, A.; DEEB, A. "Synthesis and reaction of 3-cyano 2-(1H)-pyridones."<br>page 497, column 1, abstract no. 137633m<br>& Pak. J. Sci. Ind. Res. 1977, 20(3), 139-49<br>-- | 1 | TECHNICAL FIELDS SEARCHED (Int. Cl.4)<br>C 07 D 405/00<br>C 07 D 493/00 |
| A | CHEMICAL ABSTRACTS, vol. 104, no. 15, April 14, 1986, Columbus, Ohio, USA<br>MOHAMED, M.M.; EL-HASHASH, M. A.; SAYED, M. A.; SHEHATA, A.A. "Reactions of some 2(1H)-pyridones synthesized from 3,4-methylene-dioxybenzal and 4-nitrobenzal-p-isopropylacetophenone."<br>page 689, column 1, abstract no. 129774a<br>& J. Chem. Soc. Pak. 1985, 7(1), 47-58<br>-- | 1 | |

The present search report has been drawn up for all claims

| Place of search | Date of completion of the search | Examiner |
|---|---|---|
| VIENNA | 27-07-1987 | HAMMER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0247721

Application number

European Patent Office

# EUROPEAN SEARCH REPORT

EP 87303486.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 3 862 952 (MARKLEY)<br>* Abstract *<br>---- | 1 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 27-07-1987 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document